# EUROPEAN PATENT SPECIFICATION

(11) **EP 2 255 764 B1**
(45) Date of publication and mention of the grant of the patent: **11.09.2013**
(21) Application number: 09425207.9
(22) Date of filing: 26.05.2009
(51) Int. Cl.: A61F 13/49, A61F 13/56, A61F 13/15

(54) **A side panel for sanitary articles, corresponding method of manufacture and article**
Seitenwand für Sanitärartikel, entsprechendes Herstellungsverfahren und Artikel
Panneau latéral pour articles sanitaires, procédé correspondant de fabrication et article

(43) Date of publication of application: 01.12.2010
(73) Proprietor: Fameccanica. Data S.p.A., 66020 Sambuceto di S. Giovanni Teatino (Chieti) (IT)
(72) Inventor: Sablone, Gabriele, 66015 Montesilvano (PE) (IT); Pasqualoni, Paolo, 66020 Sambuceto di S. Giovanni Teatino (CH) (IT)
(74) Representative: Bosotti, Luciano

(56) References cited:
- WO-A-2007/071267
- DE-U1- 9 422 298
- US-A- 4 317 449
- US-A- 5 531 731
- US-A1- 2005 048 303
- US-A1- 2006 271 004

## Description

### Field of the invention

The present disclosure refers to the sanitary products industry.

In particular, the disclosure refers to the so-called side panels for sanitary products wearable like briefs.

### Description of the related art

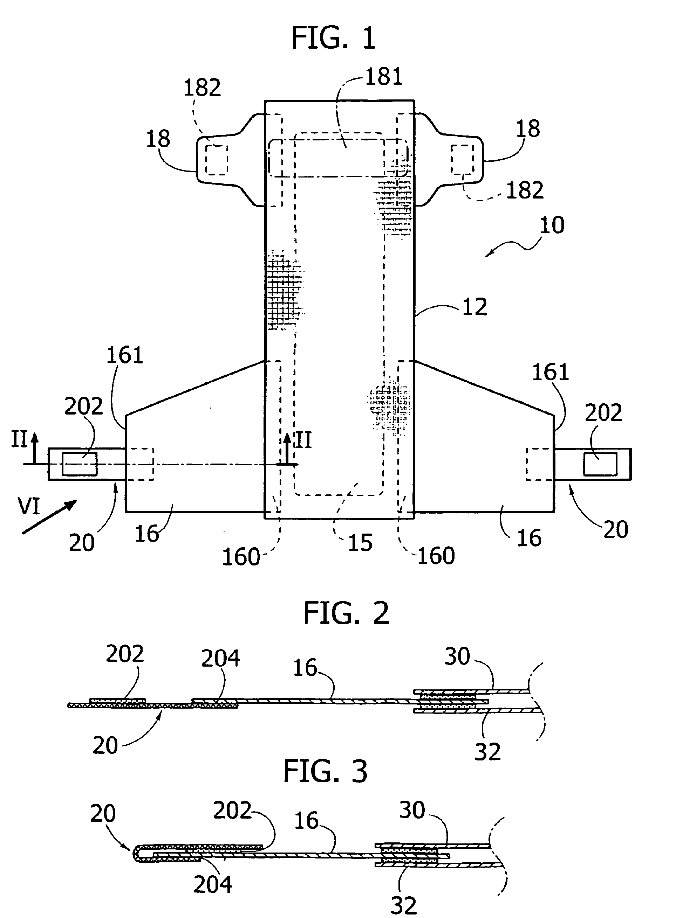

In the field of sanitary items wearable like pants (diapers for newborns and children, incontinence pads for adults, etc...), use of the structure generally represented in figure 1, where the article of the considered type is represented in an unfolded form, has become more and more consolidated.

Such an article, indicated in its entirety with 10, includes a chassis 12 intended to be arranged according to a general concave configuration around the crotch region of the user. The chassis 12 includes an absorbent core 15 enclosed sandwich-like between a topsheet permeable to body liquids and a backsheet impermeable to such liquids.

In order to allow the article 10 to be worn like pants, provided for fastening it around the waistline of the user are the so called side panels, i.e. laminar bodies - respectively rear 16 and front 18 - connectable in closed conditions around the waistline of the user on one side and the other of the article 10 at their distal margins through elements 20.

As known to those skilled in the art, a wide range of solutions have been developed around this base over the years. These solutions regard, for example, the possibility of including in the chassis 12 elements such as longitudinal and transverse elastic bands, cuffs intended to counter leakage of body discharges from the collection region at the core 15, additional layers, such as so-called "acquisition layers" etc...

Analogous considerations are valid also regarding the side panels, that confer to the absorbent product the characteristic hourglass configuration.

Initially, the abovementioned panels used to be obtained by extending the topsheet and the backsheet sideways, coupling them together using adhesives (and/or through known techniques such as thermal bonding, ultrasonic bonding) and removing from each of the two sides of the absorbent core a portion of said material coupled by means of a curved cut-out obtained between the front region, the rear region and the inner edge delimited by the longitudinal elastication strips.

The latest developments of the product have led to reducing the width of the topsheet and of the backsheet and the hourglass shape has been replicated by applying rectangular panels or preferably shaped in proximity to the distal edges of the chassis 12.

The abovementioned side panels may also be present at only one of the ends of the chassis 12, for example avoiding the presence of side panels 18 represented in figure 1.

In the most common embodiments, the closing elements 20 are arranged on the side panels 16 located at the rear end of the chassis 12 (intended to be set facing the back of the user). However, at least generally speaking, they could also be arranged on the side panels 18 located at the front end of the chassis 12 (intended to be set facing the stomach of the user), or equally on panels 16, or panels 18, i.e. to both ends of the chassis 12. For the sake of simplicity, the present description shall refer to embodiments in which the closing elements 20 are arranged (only) on the side panels 16 located at the rear end of the chassis 12.

The side panels are a distinguishing characteristic of the latest articles 10.

Regarding the provision of side panels, the solution concerning use of elastic material, such to confer the article 10 good "wearability" characteristics with respect to the body of the user has become more and more consolidated over time. Particularly appreciated by the public are the embodiments that refer, within the general development of the article 10, to the overall hourglass shape and which have an anatomic configuration.

Regarding the closing elements 20, alongside the traditional adhesive fixing solutions, the solutions providing for a hook-and-loop closing system have become more and more common.

This system may provide for a single element 181 (referred to as frontal tape, whose profile is represented with a dot and dash line in figure 1) applied on the front part of the chassis 12 or two separate elements 182 (whose profiles are represented with a dashed line in figure 1) applied on the front part of the front panels 18. The elements 181 or 182 bear the loops of the hooking system, while closing elements 20 represented in figure 1 are formed by labels projecting from the distal edges of the side panels 16 and which bear a hook strip 202 intended to be coupled with the loops of the elements 181 or 182.

The view of figure 1 is conceptually applied both to prior art solutions and embodiments of the solution described herein.

The views of figures 2 and 3 correspond to an ideal sectional view according to line II-II of figure 1 and represent - with reference to solutions known and represented in simplified form - the closing element 20 respectively in unfolded position (figure 2) and in folded position on the side panel 16 (figure 3).

In the unfolded position, the elements 20 project beyond the distal edge 161 of the respective side panel 16 in the condition that allows its coupling with the loop formations present (still with reference to figure 1) in element 181 or in elements 182. The elements 181 or 182 may have a larger surface extension with respect to the formations 202 in such a manner to allow a more accurate adjustment of the closed condition of the article 10 around the body of the user, preventing the article 10 from being either too tight or too loose.

For immediate reference, it shall be observed that, with reference to the perspective of figure 1, the formations with the hooks 202 face the person observing the figure, while the formations 181 or 182 provided with loops are located on the opposite side with respect to the person observing.

At least generally speaking, the arrangement of the hooks and the respective loops could be inverted, with the loops representing the formations 202 and the hooks representing the formations 181, 182.

Furthermore, as already mentioned, the formations 202 could simply be adhesive formations intended to be adhesively coupled with the front face of the panels 18 or of the chassis 12.

In the closed condition represented in figure 3 (i.e. in the condition in which the element 202 is found when the article 10 is packaged and when the article 10 is extracted from its packaging) the element 20 "embraces" the distal edge 161 of the panel 16 and the formation 202 is set facing the body of the panel 16.

The representation of figures 2 and 3 also highlights the fact that the proximal margin 160 of the side panels 16 is usually anchored to the chassis 12 arranging it sandwich-like between the topsheet 30 and the backsheet 32 providing a stable connection, typically by means of gluing. Obviously, different fixing methods are possible and known to the art.

In order to obtain a configuration like the one illustrated in figures 2 and 3 it is possible to proceed cutting the panels 16 (for example trapezoidal-shaped, with sides more or less curved) with a shaping operation suitable to be carried out after having applied the closing element 20 onto the material intended to form the panel. As a matter of fact, it is difficult to think of folding the closing element 20 according to the general loop configuration represented in figure 3 on a web having discontinuity.

This method of operating may lead to a given waste of material, capable of reaching up to 40-50% of the total. This fact is capable of having a negative impact especially for side panels 16 made using elastic material, implying quite high costs.

The manufacturers in the industry are thus keen on solutions capable of reducing if not eliminating waste of materials of this type. Regarding this, it is possible to exploit, for example, the solution described in EP-A-1 941 853.

Equally known is the possibility of applying the closing formation 202 (by way of example one might think of a hook formation) directly on the material of the panel 16 (and not, as represented in figure 1, on a label intended to project with respect to the distal edge of the panel 16). This solution has the advantage of allowing saving material, reducing to the minimum if not entirely eliminating the wastage, in a relatively simple production process (see, for example, EP-A-0 737 057).

However, the inventors observed that this solution has the drawback linked to the fact of applying the material intended for closing (for example the hook formation 202) directly on the elastic material of the panel 16. This hinders, and actually does not allow, obtaining a flat configuration by the closing element. If applied on an elastic material, it always tends to acquire a curved configuration, which complicates the coupling, for example, of the hook formations with the complementary loop formations provided at the opposite end of the article.

Additionally, the inventors observed that this solution leads to using - for the panels 16 - a larger strip of material (elastic) with respect to the equivalent panel made according to the solution represented in figure 1 i.e. with the formation 202 on a label projecting from the distal margin of the panel 16.

Actually, if one wishes to maintain the same "useable" width of the side panel, the width of the material on which the closing formation 202 is applied must be, so to speak, "replaced" by an equivalent portion of material of the panel 16 and this may actually jeopardise the saving of material linked to the absence of wastage.

DE-U1-9422298 discloses a method for forming multiple adhesive fasteners from a continuous substrate web, wherein an adhesive layer is applied to the substrate, long stiffening webs are attached close to the edges to form a composite substrate, and the web is then separated along a line to form opposed pairs of fastener tab subassemblies.

### Object and summary of the invention

In the light of the information provided above, there arises the need to provide side panels for sanitary items of the type outlined above capable of:
- maintaining the advantages linked to the base solution represented in figure 1, in particular regarding the possibility of providing a connection formation on an element intended to project beyond the distal margin of the side panel, and
- overcome the drawbacks linked, for example, to the impossibility of having connection formations capable of maintaining a flat configuration or to the fact of requiring the use of larger amounts of material for the side panels.

The object of the invention is that of meeting such need.

According to the present invention, such object is attained by a side panel according to claim 1 and by a manufacturing method according to claim 13.

The claims form an integral part of the technical disclosure provided herein in relation to the invention.

### Brief description of the attached representations

Now, the invention shall be described, strictly for exemplifying and non-limiting purposes, with reference to the attached representations, wherein:
- figures 1 - 3 have already been described above,
- figures 4 and 5 illustrate, for direct comparison with figures 2 and 3 an embodiment described herein,
- figure 6 is a general perspective view of the embodiment of figures 4 and 5,
- figures 7, 8 and 9 illustrate, for direct comparison with figures 4 and 5 an embodiment described herein,
- figure 9 illustrates an embodiment described herein,
- figures 10, 11 and 12 represent methods for implementing the solution described herein, with figure 12 approximately corresponding to a section according to line XII-XII of figure 11, and
- figures 13 and 14 illustrate embodiments of a side panel as described herein.

### Detailed description of the embodiments

Illustrated in the following description are various specific details aimed at providing an in-depth understanding of the embodiments. The embodiments may be obtained without one or more specific details, or through other methods, components, materials etc. In other cases, known structures, materials or operations are not shown or described in detail to avoid obscuring the various aspects of the embodiments.

Reference to "an embodiment" in this description indicates that a particular configuration, structure or characteristic described regarding the embodiment is included in at least one embodiment. Hence, expressions such as "in an embodiment", possibly present in various parts of this description do not necessarily refer to the same embodiment. Furthermore, particular configurations, structures or characteristics may be combined in any suitable manner in one or more embodiments.

References herein are used for facilitating the reader and thus they do not define the scope of protection or the scope of the embodiments.

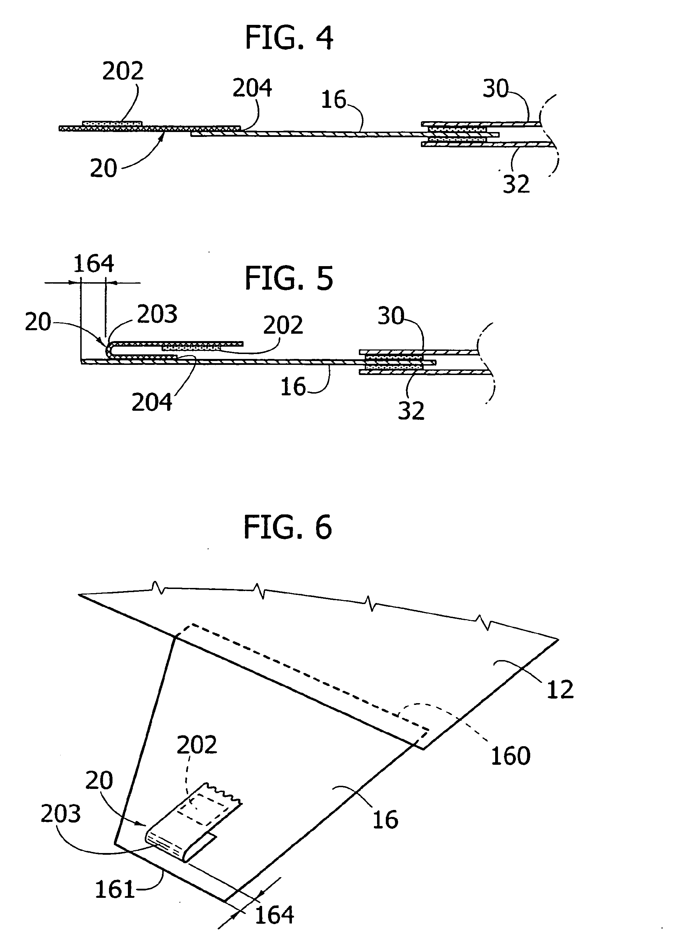

In figures 4 - 14, elements, parts and components identical or equivalent to elements, parts and components described previously with reference to figures 1 - 3 were indicated using the same reference numbers and shall not be described further herein for the sake of brevity. As already mentioned, the view of figure 1, and - unless described otherwise hereinafter
- the respective detail characteristics are applied both to prior art solutions and to the embodiment of the innovative solution subject of the present application.

Figures 4 and 5 as well as figures 7 and 8, thus correspond, the same applying to the views of figures 2 and 3, to an ideal sectional view according to line II-II of figure 1.

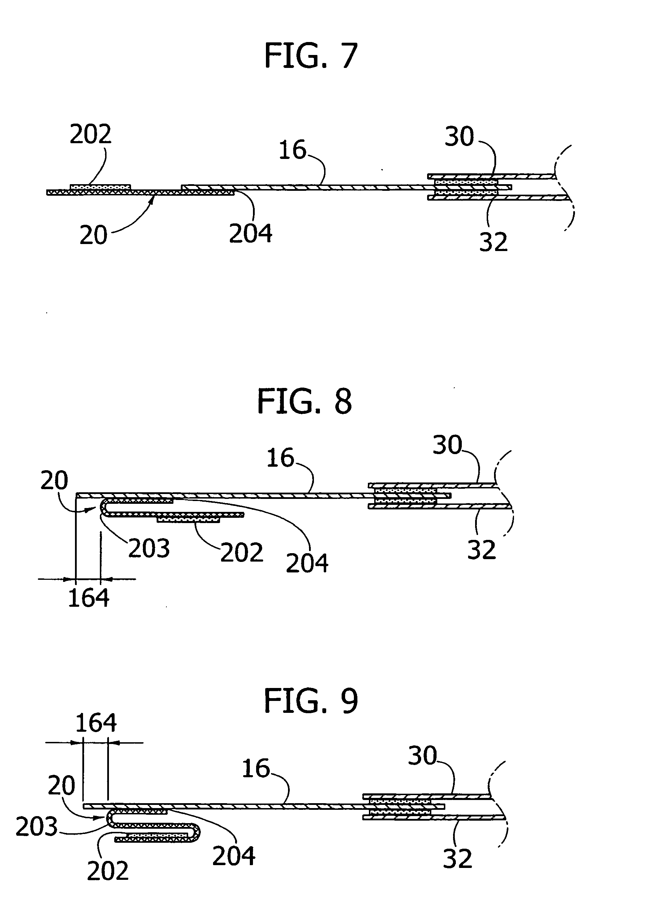

Figures 4 and 5 as well as figures 7 and 8 represent different methods of implementation and application of the elements 20 according to embodiments considered herein. The view of figure 9, which again illustrates an embodiment considered herein, substantially corresponds, according to the perspective and the illustrated condition, to figures 3, 5 and 8.

For direct comparison with figure 2, it shall also be observed that the embodiments according to figures 4 and 5 and according to figures 7 and 8 (and 9) lead - with the element 20 extended and projecting with respect to the body of the side panel 16, to having the closing formation 202 projecting on the "inner" side of the panel 16 and of the article 10, i.e. on the side - characterised by the presence of the topsheet 30 - intended to be set facing the body of the user.

As a matter of fact, the arrangement or orientation of the closing formation 202 allows to unambiguously identify the "inner" side of the panel 16 even before the latter is integrated in the article 10: examining the panel 16, even alone, it shall actually be observed that its inner side or face or surface is the one on which, with the element 20 unfolded or open, the formation 202 is found.

In addition, for immediate reference it shall be observed that, in all the embodiments considered herein, with reference to the point of view of figure 1, the formations 202 (for example with hooks) are set facing the person observing the figure, while the elements 181 or 182 (for example provided with loops) are located on the opposite side with respect to the observer. At least generally, the arrangement of the hooks and the respective loops could be inverted, with the loops representing the formations 202 and the hooks representing the elements 181 or 182.

In various embodiments, the formations 182 could simply be made up of the material itself with which the side panels 18 and/or the backsheet 32 are made in case the latter is obtained by coupling an impermeable film with a non-woven fabric in such a manner that the absorbent product may confer to the user the feeling of wearing a textile product. In this case, the fibrous structure of the non-woven fabric replaces the loops of the specific material referred to as loop. An example of material suitable for such purpose is Non Woven SPUNTEN 25 gsm PHOB made by Fiberweb located in Trezzano sul Naviglio (Milan). Furthermore, as already mentioned, the formations 202 could simply be adhesive formations intended to be adhesively coupled with the front face of the panels 18 or of the chassis 12.

Analogously to figures 2 and 3, also the embodiments of figures 4 - 9 provide for that the element 20 be arranged and fixed - for example by gluing (in 204) - on the distal edge 161 of the side panel 16, whose proximal edge 160 is instead fixed to the chassis 12, for example inserting it sandwich-like between the topsheet 30 and the backsheet 32.

Still analogously to the illustration outlined in figures 2 and 3, when arranged in closed condition (figure 5), also the closing element 20 of figure 4 and 5 has a general U-shaped configuration with the connection part 202 facing inwards the U-shaped formation (thus against the side panel 16).

The U-shaped configuration in question may be a symmetric or asymmetric configuration, for example with the branch of the U-shape intended to be adhesively connected (in 204) on the side panel 16, shorter with respect to the other branch of the U-shape, the one that leads to the closing formation 202. This dissymmetry of the U-shaped configuration does not represent an essential characteristic for the embodiments considered herein.

However, considering the differences between the solution illustrated in figures 2 and 3 and the embodiment of figures 4 and 5 it shall be observed that, while in the solution of figures 2 and 3 the connection of the element 20 to the distal edge of the side panel 16 is attained on the "outer" side of the panel 16 (i.e. on the side intended to be set facing outwards the article 10 when worn i.e. on the side where the backsheet 32 is found) in the embodiment of figures 4 and 5 such connection (though other connection methods, such as for example thermal bonding or ultrasonic bonding are possible, reference shall still be made to an adhesive connection for the sake of simplicity) is located on the inner side of the side panel 16 i.e. on the side intended to face towards the body of the user (inner side characterised by the presence of the topsheet 30).

In the various embodiments described herein it is further provided for that the vertex 203 of the U-shaped element be entirely arranged on the panel 16 in such a manner that present between the distal edge 161 of the panel 16 and the vertex 203 of the U-shaped configuration of the closing element 20 there be a distance 164.

In various embodiments, such distance 164 may vary from a minimum of 0.1 mm to a maximum of 10 mm, with a preferred distance ranging from a minimum of 3 mm to a maximum of 5 mm.

Figure 6 illustrates a perspective representation of the embodiment represented in figures 4 and 5 fully observable from which is the fact that in the embodiment of figures 4 - 6, the closing element 20, in its entirety, is fully found on the inner side of the elastic panel 16 and not, as it occurs in the solution represented in figures 2 and 3, astride the distal edge of the panel 16 itself.

The fact of not having to arrange the element 20 astride the distal edge of the side panel 16 represents a simplification of the manufacturing method, in the terms that shall be more apparent hereinafter.

The same criterion, i.e. that of obtaining the element 20 in such a manner that the same is entirely found on a side of the side panel 16 (and not astride the same, in the closed condition) is also reutilised in the embodiment of figures 7 and 8 which, once again represent the closing element 20:
- in the unfolded or open condition, of use (figure 7, analogously to figures 2 and 4) and,
- in the closed condition, used in the packaging step (figure 8, analogously to figures 3 and 5).

In particular, in the embodiment of figures 7 and 8, the connection region 204 (also in this case an adhesive, through thermal bonding, ultrasonic bonding, etc...) between the distal edge of the side panel 16 and the closing element 20 is located on the outer side of the panel 16 (i.e. on the side intended to be set facing outwards with respect to the user, i.e. on the side where the backsheet 32 is found) and, in the U-shaped closed condition (figure 8), the connection formation 202 is found on the outer side of the U-shape.

The embodiment of figures 7 and 8 leads to having a connection region 204, for example when it is attained in adhesive form, to be located on the outer surface of the panel 16, exposed towards the garments of the user. This solution is better observable, at least in some conditions of use.

Even in this case, present between the distal edge 161 of the panel 16 and the vertex 203 of the U-shape of the closing element 20 is the distance 164.

The same also applies for figure 9, which illustrates an embodiment still referable to the general concept of arranging the closing element 20 entirely on one side of the panel 16.

The embodiment of figure 9 (which in the unfolded condition of the element 20 reproduces the situation represented in figure 7 in an almost identical manner) refers to the embodiment of figures 7 and 8 regarding the arrangement of the connection zone 204 on the "outer" side of the distal edge of the panel 16.

However, the embodiment of figure 9 provides for that, in a closed or folded condition, the longer branch of the U-shape, the one bearing the closing formation 202, be folded further in such a manner to lead the formation 202 - in the folded condition of the element 20 - to be set facing the panel 16 (and not arranged facing away from the same as represented in figure 8).

In other words, in the embodiment represented in figure 9 the element 20 is folded according to a general S-shape wherein one can distinguish:
- a proximal section, connected - in 204 - to the panel 16,
- an intermediate section, and
- a distal section that bears the formation 202 set facing the intermediate section.

All the embodiments of figures 5 - 9 (whose specific characteristics are freely transferable from an embodiment to another), have in common:
- the fact of obtaining - in the unfolded condition (figure 4 and figure 7, the latter applicable also to the solution represented in figure 9) - an operative configuration substantially analogous to the one represented in figure 1,
- the possibility of applying the element 20 in closed condition (figure 5, figure 8 and figure 9) operating only on one side of the panel 16, and
- the fact of having a distance 164 between the distal edge 161 of the panel 16 and the vertex 203 of the folded configuration of the element 20.

This operation mode may be advantageous in various process situations, in particular using high speed application lines according to the methods similar to those described in EP-A- 1 941 853, where - in detail - a closing element 20 as outlined in the present document may already be applied in the folded configuration, thus not requiring, passing through a special unit that folds the single closing systems 20 on the panel 16: a man skilled in the art shall observe the fact that folding the element 20 when it is still a continuous strip is technically much easier and efficient.

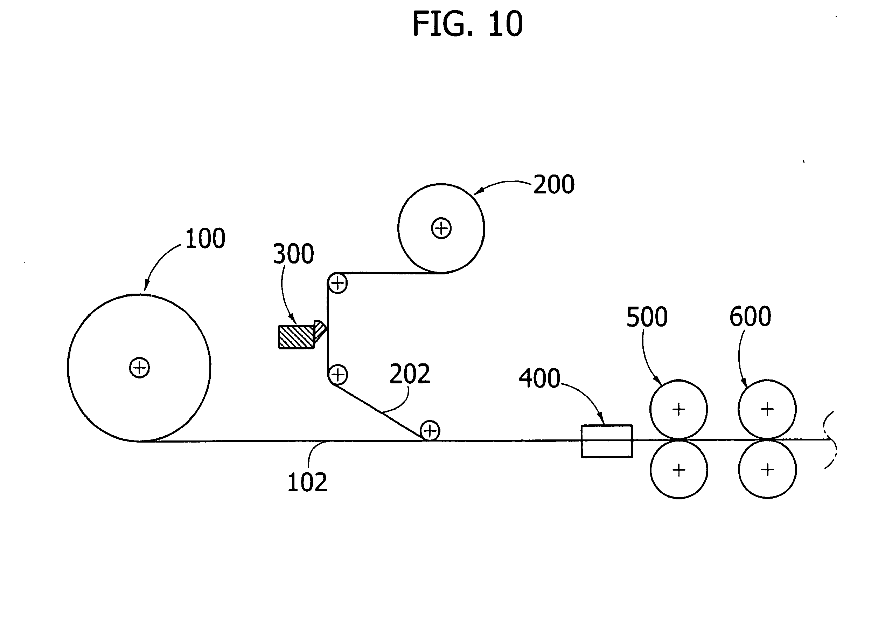

In figure 10, regarding a possible method for obtaining the side panels 16 of the type described herein, the references 100 and 200 respectively indicate the sources from which the following unwind: a strip made of a first material 102 and two strips made of a second material 202 intended to obtain the body of the fixing elements 20 and the closing formations 202, respectively.

For example, the material unwound from the reel 100 may be a strip of non-woven fabric. The non-woven material PP type SMS with a basis weight of 50gr/m² produced by various manufacturers such as Fiberweb in Trezzano sul Naviglio (Milan), Albis in Cuneo or Atex in Settala (Milan) is exemplary of non-woven material useable in the context illustrated herein.

The material unwound from the two reels 200 arranged paired may be, for example, a material of the hook type suitable to cooperate with a complementary material of the loop type to obtain a closure of the hook-and-loop type. A material useable in the present contest is the hook material manufactured by Binder GmbH in Tuttlingen (Germany).

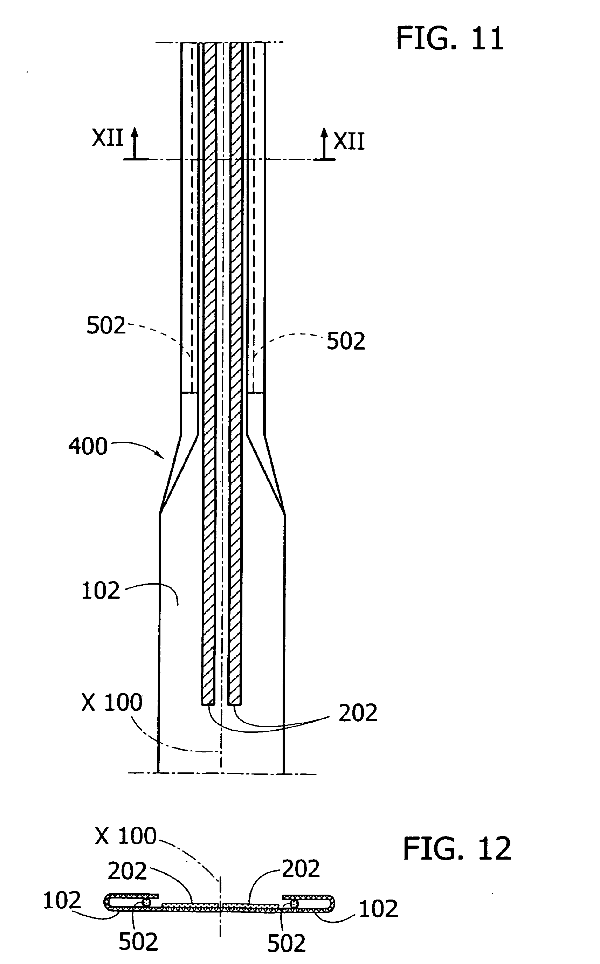

In an embodiment, as better observable in the view of figure 12, the method provides for the simultaneous obtainment of two symmetric strips obtained from which may be two elements 20 of the type described previously.

For such purpose, as better observable in the view of figure 11, the material that is unwound from the reel 100 (indicated with 102 also in figure 11 for the sake simplicity) receives starting from the two reels 200 two adjacent strips (also indicated with 202 in figure 11 for the sake of simplicity) of hook material.

The connection of the two strips or bands 202 on the strip 100 is obtained, for example, by adhesive means through glue spread on the back of the strips 202 (the term back is used to indicate the generally flat part, opposite to the one on which hooks are provided for) applied on the strip 202 in a station for applying the glue 300 interposed in the path for unwinding the strips. A glue suitable for use in the illustrated contest is the glue available as PM17 manufactured by Savaré Specialty Adhesives in Milan.

The composite strip formed by the non-woven material 102 with the two strips of hook material 202 applied adjacent to each other in proximity to the centreline X100 of the strip 102 itself is then subjected - in a folding station 400 - to an operation of folding the edges (performable, for example, according to the criteria illustrated in EP-A-0 638 505) in such a manner to generate two strip parts folded to form a U-shape, symmetric with respect to each other, as represented in figure 12. Each of these two strip parts is folded to form a U-shape according to a configuration analogous to that of the element 20 represented in figure 5: for immediate reference, number 20 corresponds to number 102 and the adhesive connection of the formation 202 on the material 20 corresponds to the glue applied in the station 300.

All this observing that:
- the strip exiting from the folding station 400 includes two U-shapes (as represented in figure 5) connected to each other at the distal margin of the longer arm of the U-shape, and
- criteria entirely analogous to that represented in figures 10 - 12 may be applied to obtain the U-shape represented in figure 8 and, except for a more complex folding configuration, the S-shaped or Z-shaped formation represented in figure 9.

In the illustrated embodiment, the composite strip 102/202 exiting from the folding station 400 passes through a bonding unit 500 intended to create a temporary bonding line 502 between the two branches of the U-shape sufficiently to allow each of the two U-shapes to remain closed during the subsequent treatment operations up to the packaging of the product. Such connection line 502 is sufficiently thin and weak to open easily when the product is opened by the user who thus proceeds to unfold the element 20 from the folded configurations represented in figure 5, in figure 8 and in figure 9 towards the unfolded configurations represented in figure 4 and in figure 7.

The station 500 may be represented, for example, by a "tacking" station operating via ultrasonic thermal bonding i.e. by applying a so-called "green" glue.

In addition, present downstream of the station 500 is a cutting station 600 intended to divide the folded composite strip 102/202 described previously in such a manner to obtain two strips each of which is suitable to be segmented in such a manner to obtain single elements 20 as represented, for example, in figure 6.

The abovementioned segmentation operation may be either carried out in a single production process or according to an "off-line" operation mode, such to provide for that the composite strip 102/202 before or after the longitudinal cutting operation along line X100 be wound in reels and stored. All this for subsequently using the storage reels for feeding the steps for manufacturing the actual panels 16.

According to such criteria, the single elements 20 bearing the closing formations 202 obtained through the longitudinal cutting and the segmentation of the composite strip 102/202 described previously are applied at predetermined distances on a strip of material 1000 adapted for manufacturing the side panels 16.

In particular figure 12 refers to the application of elements 20 according to a symmetric arrangement that provides for the formation of two adjacent rows of elements 20 with a predetermined pitch on the strip 1000.

Such application provides for the connection of the elements 20 to the material 1000 with the application, for example, of adhesive regions (or through the formation of connection regions of another type: thermal bonding, ultrasonic bonding, etc..., already mentioned previously) intended to form the connection regions 204 represented in figures 4, 5 and 7- 9 .

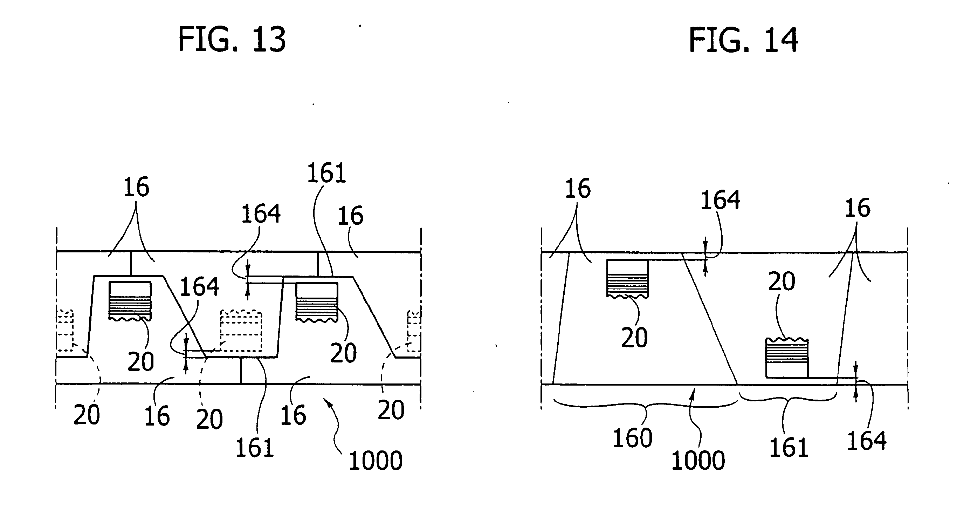

The strip 1000 is then subjected to segmentation or according to the zig zag profile represented with a continuous line in figure 13 or simply through oblique cuttings as represented in figure 14 in such a manner to obtain single side panels.

The latter are then applied on an article such as the article 10 of figure 1 according to the methods described more in detail in EP-A-1 941 853 already mentioned several times previously.

In any case, such techniques obtain advantage from the fact that, in the embodiments exemplified herein, the closing element 20 is connected (in 204) to one of the surfaces of the side panel 16 and, in closed condition, it is folded adjacent to such surface of the side panel 16.

In various embodiments (for example figure 5 and 8) in the abovementioned closed condition, the closing element 20 is folded according to a general loop condition with a branch of the loop connected (in 204) to the side panel 16 and the other branch of the loop bearing the closing formation 202.

The closing element 20 may be applied (figure 4-6 ) on the internal surface of the panel 16, intended to face towards the body of the user with the closing formation 202 applied inside the loop configuration.

The closing element 20 may be applied (figure 7 and 8) on the external surface of the panel 16, intended to be arranged opposite to the body of the user, i.e. facing outwards. The closing formation 202 may be applied on the interior of the loop configuration.

In various embodiments the loop configuration includes a smaller branch connected to the side panel 16 and a larger branch bearing the closing formation 202.

The loop configuration may be a U-shaped configuration, or a configuration in which a larger branch of the loop formation is in turn folded to form a U-shape, conferring to the closing element 20 a general S-shaped configuration with the closing formation 202 in concealed position.

Hence, without prejudice to the underlying principle of the invention, the details and embodiments may vary, even significantly, with respect to what has been described herein by way of non-limiting example only, without departing from the scope of the invention as defined by the attached claims.

## Claims

1. A side panel (16) for a pant-like sanitary article (10) having a chassis (12), said side panel (16) including:
- a laminar body having two opposite surfaces, a proximal edge (160) and a distal edge (161), the proximal edge (160) being intended to be fixed to the chassis (12) of said sanitary article (10),
- a closing element (20) having a closing formation (202), the closing element (20) being fixed to one of said surfaces of said laminar body in proximity to said distal edge (161), the closing element (20) having a closed condition corresponding to the packaged condition of the sanitary article (10) and a condition of use, wherein in said closed condition the closing element (20) is folded adjacent to said laminar body and in said condition of use the closing element (20) is unfolded and projects from said distal edge (161) of said laminar body, wherein in said closed condition, said closing element (20) is folded against said one of said surfaces of said laminar body (16), **characterised in that**, in said closed condition, said closing element (20) is located at a distance (164) from the distal edge (161) of the laminar body.

2. The panel according to claim 1, wherein said distance (164) is between 0.1 mm and 10 mm, preferably between 3 mm and 5mm.

3. The panel according to any one of the preceding claims, wherein, in said closed condition, said closing element (20) is folded according to a loop configuration with a branch of said loop connected (204) to said one of said surfaces of the side panel (16) and the other branch of the loop bearing said closing formation (202).

4. The panel according to claim 3, wherein said closing element (20) is applied on the inner surface of said panel (16) intended to face towards the body of the user, with said closing formation (202) applied inside said loop configuration.

5. The panel according to claim 3, wherein said closing element (20) is applied on the outer surface of said side panel (16) intended to be opposite with respect to the body of the user, with said closing formation (202) applied outside said loop configuration.

6. The panel according to any one of claims 3 - 5, wherein said loop configuration includes a shorter branch (204) connected to said one of said surfaces of the side panel (16) and a longer branch bearing said closing formation (202).

7. The panel according to any one of claims 3 - 6, wherein said loop configuration is a U-shaped configuration.

8. The panel according to claim 6, wherein said longer branch of said loop formation is in turn folded to form a U-shape, conferring to said closing element (20) in said closed condition a general S-shaped configuration with said closing formation (202) in a concealed position.

9. The panel according to any one of the preceding claims, wherein said closing element (20) is connected (204) to said side panel (16) by adhesive means, through thermal bonding or through ultrasonic bonding.

10. The panel according to any one of the preceding claims, wherein said closing formation (202) is an adhesive formation.

11. The panel according to any one of claims 1 - 9, wherein said closing formation (202) is one of the parts of a hook-and-loop closure.

12. The panel according to claim 11, wherein said closing formation (202) is the hook part of a hook-and-loop closure.

13. A method of producing side panels (16) for a pant-like sanitary article (10), including the steps of:
- providing a strip (1000),
- providing a plurality of closing elements (20) in a folded closed condition, the closing elements having respective closing formations (202),
- fixing the closing elements (20) in said closed condition to said strip (1000),
- cutting the strip (1000) with the closing elements (20) fixed thereto so as to provide a plurality of side panels (16) including laminar bodies having proximal edges (160) and distal edges (161) and respective closing elements (20), wherein said closing elements (20), in said closed condition, are fixed to one surface of the respective laminar bodies in proximity to said distal edges (161), are located at a distance (164) from said distal edges (161) of the respective laminar bodies and are folded against said one surface of said laminar bodies (16), the closing elements being adapted to be unfolded to a condition of use in which the closing elements (20) project from said distal edges (161) of said laminar bodies.

14. The method according to claim 13, wherein the step of providing a plurality of closing elements (20) in a folded closed condition, includes the steps of:
- providing a first strip (102) of laminate material for obtaining said closing elements, said first strip having a centreline (X100),
- providing two second strips (202) of material for obtaining said closing formation,
- connecting said two second strips (202) on said first strip (102) on opposite sides of the centreline (X100), and
- folding the external edges of said first strip (102) towards said centreline (X100) in such a manner to produce a composite strip including two branches symmetric with respect to said centreline (X100), each branch having a transverse loop profile and being segmentable to obtain said closing elements (20), each applicable on a side panel (16) in said closed condition.

15. A sanitary article (10) wearable like pants including:
- a chassis (12) to be arranged concave around the body of the user and having a front end and a rear end, and
- a pair of side panels (16) according to any one of claims 1 - 12 applied symmetrically on opposite sides of said chassis (12) in correspondence with at least one of the front and rear ends of the article (10).

## Patentansprüche

1. Seitenwand (16) für einen hosenähnlichen Sanitärartikel (10) mit einem Rahmen (12), wobei die Seitenwand (16) umfasst:
- einen flächigen Hauptteil mit zwei gegenüberliegenden Flächen, einer proximalen Kante (160) und einer distalen Kante (161), wobei die proximale Kante (160) dazu bestimmt ist, am Rahmen (12) des Sanitärartikels (10) befestigt zu werden,
- ein Schließelement (20) mit einer Schließformation (202), wobei das Schließelement (20) an einer der Flächen des flächigen Hauptteils in unmittelbarer Nähe zur distalen Kante (161) befestigt ist, das Schließelement (20) einen geschlossenen Zustand, der dem verpackten Zustand des Sanitärartikels (10) entspricht, und einen Gebrauchszustand aufweist, wobei das Schließelement (20) im geschlossenen Zustand benachbart zum flächigen Hauptteil umgeklappt ist, und das Schließelement (20) im Gebrauchszustand aufgeklappt ist und von der distalen Kante (161) des flächigen Hauptteils vorsteht, wobei das Schließelement (20) im geschlossenen Zustand gegen eine der Flächen des flächigen Hauptteils (16) umgeklappt ist,
**dadurch gekennzeichnet, dass** das Schließelement (20) im geschlossenen Zustand in einem Abstand (164) von der distalen Kante (161) des flächigen Hauptteils angeordnet ist.

2. Wand nach Anspruch 1, wobei der Abstand (164) 0,1 mm bis 10 mm, vorzugsweise 3 mm bis 5 mm beträgt.

3. Wand nach einem der vorhergehenden Ansprüche, wobei das Schließelement (20) gemäß einer Schlaufenkonfiguration umgeklappt ist, wobei ein Zweig der Schlaufe (204) mit der einen der Flächen der Seitenwand (16) verbunden ist, und der andere Zweig der Schlaufe die Schließformation (202) trägt.

4. Wand nach Anspruch 3, wobei das Schließelement (20) auf der Innenfläche der Wand (16) angebracht ist, die dem Körper des Benutzers zugewandt sein soll, wobei die Schließformation (202) innerhalb der Schlaufenkonfiguration angebracht ist.

5. Wand nach Anspruch 3, wobei das Schließelement (20) auf der Außenfläche der Seitenwand (16) angebracht ist, die in Bezug auf den Körper des Benutzers gegenüberliegend sein soll, wobei die Schließformation (202) außerhalb der Schlaufenkonfiguration angebracht ist.

6. Wand nach einem der Ansprüche 3 bis 5, wobei die Schlaufenkonfiguration einen kürzeren Zweig (204), der mit der einen der Flächen der Seitenwand (16) verbunden ist, und einen längeren Zweig umfasst, der die Schließformation (202) trägt.

7. Wand nach einem der Ansprüche 3 bis 6, wobei die Schlaufenkonfiguration eine U-förmige Konfiguration ist.

8. Wand nach Anspruch 6, wobei der längere Zweig der Schlaufenformation abwechselnd umgeklappt ist, um eine U-Form zu bilden, die dem Schließelement (20) im geschlossenen Zustand eine im Allgemeinen S-förmige Konfiguration verleiht, wobei die Schließformation (202) in einer verborgenen Position ist.

9. Wand nach einem der vorhergehenden Ansprüche, wobei das Schließelement (20) durch Klebemittel, durch Wärmebindung oder durch Ultraschallbindung mit der Seitenwand (16) verbunden ist (204) .

10. Wand nach einem der vorhergehenden Ansprüche, wobei die Schließformation (202) eine Klebeformation ist.

11. Wand nach einem der Ansprüche 1 bis 9, wobei die Schließformation (202) einer der Teile eines Haken- und Ösen-Verschlusses ist.

12. Wand nach einem der Ansprüche 1 bis 9, wobei die Schließformation (202) der Hakenteil des Haken- und Ösen-Verschlusses ist.

13. Verfahren zur Herstellung von Seitenwänden (16) für einen hosenähnlichen Sanitärartikel (10), umfassend die folgenden Schritte:
- Bereitstellen eines Streifens (1000),
- Bereitstellen einer Mehrzahl von Schließelementen (20) in einem umgeklappten geschlossenen Zustand, wobei die Schließelemente jeweilige Schließformationen (202) aufweisen,
- Befestigen der Schließelemente (20) im geschlossenen Zustand am Streifen (1000),
- Zuschneiden des Streifens (1000) mit den daran befestigten Schließelementen (20), um eine Mehrzahl von Seitenwänden (16) bereitzustellen, die flächige Hauptteile mit proximalen Kanten (160) und distalen Kanten (161) und jeweilige Schließelemente (20) umfassen, wobei die Schließelemente (20) im geschlossenen Zustand an einer Fläche der jeweiligen flächigen Hauptteile in unmittelbarer Nähe zu den distalen Kanten (161) befestigt werden, in einem Abstand (164) von den distalen Kanten (161) der jeweiligen flächigen Hauptteile (16) angeordnet werden und gegen die eine Fläche der flächigen Hauptteile (16) umgeklappt werden, wobei die Schließelemente so ausgelegt sind, dass sie in einen Gebrauchszustand aufgeklappt werden, in welchem die Schließelemente (20) von den distalen Kanten (161) der flächigen Hauptteile vorstehen.

14. Verfahren nach Anspruch 13, wobei der Schritt des Bereitstellens einer Mehrzahl von Schließelementen (20) in einem umgeklappten geschlossenen Zustand die folgenden Schritte umfasst:
- Bereitstellen eines ersten Streifens (102) von flächigem Material zum Erhalten der Schließelemente, wobei der erste Streifen eine Mittellinie (X100) aufweist,
- Bereitstellen von zwei zweiten Streifen (202) von Material zum Erhalten der Schließformation,
- Verbinden der beiden zweiten Streifen (202) auf dem ersten Streifen (102) auf gegenüberliegenden Seiten der Mittellinie (X100), und
- derartiges Umklappen der Außenkanten des ersten Streifens (102) zur Mittellinie (X100), dass ein zusammengesetzter Streifen gebildet wird, der zwei Zweige symmetrisch in Bezug auf die Mittellinie (X100) umfasst, wobei jeder Zweig ein Querschlaufenprofil aufweist und segmentiert werden kann, um die Schließelemente (20) zu erhalten, die jeweils auf einer Seitenwand (16) im geschlossenen Zustand angebracht werden können.

15. Sanitärartikel (10), der wie eine Hose getragen werden kann und umfasst:
- einen Rahmen (12), der konkav um den Körper des Benutzers angeordnet werden soll und ein vorderes Ende und ein hinteres Ende aufweist, und
- ein Paar von Seitenwänden (16) nach einem der Ansprüche 1 bis 12, die symmetrisch auf gegenüberliegenden Seiten des Rahmens (12) in Entsprechung mit mindestens einem von den vorderen und hinteren Enden des Artikels (10) angebracht sind.

## Revendications

1. Panneau latéral (16) pour un article sanitaire en forme de culotte (10) ayant un châssis (12), ledit panneau latéral (16) comprenant :
un corps laminaire ayant deux surfaces opposées, un bord proximal (160) et un bord distal (161), le bord proximal (160) étant prévu pour être fixé sur le châssis (12) dudit article sanitaire (10),
un élément de fermeture (20) ayant une formation de fermeture (202), l'élément de fermeture (20) étant fixé sur l'une desdites surfaces dudit corps laminaire à proximité dudit bord distal (161), l'élément de fermeture (20) ayant une condition fermée correspondant à la condition emballée de l'article sanitaire (10) et une condition d'utilisation, dans lequel, dans ladite condition fermée, l'élément de fermeture (20) est plié de manière adjacente audit corps laminaire et dans ladite condition d'utilisation, l'élément de fermeture (20) est déplié et fait saillie dudit bord distal (161) dudit corps laminaire, dans lequel, dans ladite condition fermée, ledit élément de fermeture (20) est plié contre ladite une desdites surfaces dudit corps laminaire (16),
**caractérisé en ce que**, dans ladite condition fermée, ledit élément de fermeture (20) est positionné à une distance (164) du bord distal (161) du corps laminaire.

2. Panneau selon la revendication 1, dans lequel ladite distance (164) est comprise entre 0,1 mm et 10 mm, de préférence entre 3 mm et 5 mm.

3. Panneau selon l'une quelconque des revendications précédentes, dans lequel, dans ladite condition fermée, ledit élément de fermeture (20) est plié selon une configuration de boucle avec une branche de ladite boucle raccordée (204) à ladite une desdites surfaces du panneau latéral (16) et l'autre branche de la boucle supportant ladite formation de fermeture (202).

4. Panneau selon la revendication 3, dans lequel ledit élément de fermeture (20) est appliqué sur la surface interne dudit panneau (16) prévue pour être orientée vers le corps de l'utilisateur, avec ladite formation de fermeture (202) appliquée à l'intérieur de ladite configuration de boucle.

5. Panneau selon la revendication 3, dans lequel ledit élément de fermeture (20) est appliqué sur la surface externe dudit panneau latéral (16) prévue pour être opposée au corps de l'utilisateur, avec ladite formation de fermeture (202) appliquée à l'extérieur de ladite configuration de boucle.

6. Panneau selon l'une quelconque des revendications 3 à 5, dans lequel ladite configuration de boucle comprend une branche plus courte (204) raccordée à ladite une desdites surfaces du panneau latéral (16) et une branche plus longue supportant ladite formation de fermeture (202).

7. Panneau selon l'une quelconque des revendications 3 à 6, dans lequel ladite configuration de boucle est une configuration en forme de U.

8. Panneau selon la revendication 6, dans lequel ladite branche plus longue de ladite formation de boucle est à son tour pliée pour former une forme de U, conférant audit élément de fermeture (20) dans ladite condition fermée, une configuration générale en forme de S avec ladite formation de fermeture (202) dans une position dissimulée.

9. Panneau selon l'une quelconque des revendications précédentes, dans lequel ledit élément de fermeture (20) est raccordé (204) audit panneau (16) par des moyens adhésifs, par le biais du thermosoudage ou du soudage par ultrasons.

10. Panneau selon l'une quelconque des revendications précédentes, dans lequel ladite formation de fermeture (202) est une formation adhésive.

11. Panneau selon l'une quelconque des revendications 1 à 9, dans lequel ladite formation de fermeture (202) est l'une des parties d'une fermeture à Velcro.

12. Panneau selon la revendication 11, dans lequel ladite formation de fermeture (202) est la partie à crochets d'une fermeture à Velcro.

13. Procédé pour produire des panneaux latéraux (16) pour un article sanitaire en forme de culotte (10), comprenant les étapes consistant à :
prévoir une bande (1000),
prévoir une pluralité d'éléments de fermeture (20) dans une condition fermée pliée, les éléments de fermeture ayant des formations de fermeture (202) respectives,
fixer les éléments de fermeture (20) dans ladite condition fermée, sur ladite bande (1000),
couper la bande (1000) avec les éléments de fermeture (20) fixés sur cette dernière afin de fournir une pluralité de panneaux latéraux (16) comprenant des corps laminaires ayant des bords proximaux (160) et des bords distaux (161) et des éléments de fermeture (20) respectifs, dans lequel lesdits éléments de fermeture (20), dans ladite condition fermée, sont fixés sur une surface des corps laminaires respectifs à proximité desdits bords distaux (161), sont positionnés à une distance (164) desdits bords distaux (161) des corps laminaires respectifs et sont pliés contre ladite une surface desdits corps laminaires (16), les éléments de fermeture étant adaptés pour être dépliés dans une condition d'utilisation, dans laquelle les éléments de fermeture (20) font saillie desdits bords distaux (161) desdits corps laminaires.

14. Procédé selon la revendication 13, dans lequel l'étape consistant à prévoir une pluralité d'éléments de fermeture (20) dans une condition fermée pliée, comprend les étapes consistant à :
prévoir une première bande (102) de matériau stratifié pour obtenir lesdits élément de fermeture, ladite première bande ayant un axe central (X100),
prévoir deux secondes bandes (202) de matériau pour obtenir ladite formation de fermeture,
raccorder lesdites deux secondes bandes (202) sur ladite première bande (102) sur les bords opposés de l'axe central (X100), et
plier les bords externes de ladite première bande (102) vers ledit axe central (X100) afin de produire une bande composite comprenant deux branches symétriques par rapport audit axe central (X100), chaque branche ayant un profil de boucle transversal, et pouvant être segmentée pour obtenir lesdits éléments de fermeture (20), chacun applicable sur un panneau latéral (16) dans ladite condition fermée.

15. Article sanitaire (10) pouvant être porté comme une culotte, comprenant :
un châssis (12) destiné à être agencé de manière concave autour du corps de l'utilisateur et ayant une extrémité avant et une extrémité arrière, et
un paire de panneaux latéraux (16) selon l'une quelconque des revendications 1 à 12, appliqués symétriquement sur les côtés opposés dudit châssis (12) en correspondance avec au moins l'une parmi l'extrémité avant et l'extrémité arrière de l'article (10).
